# EUROPEAN PATENT SPECIFICATION

(11) **EP 4 129 364 B1**
(45) Date of publication and mention of the grant of the patent: **26.03.2025**
(21) Application number: 21789530.9
(22) Date of filing: 13.04.2021
(51) Int. Cl.: A61M 5/30, A61M 37/00, A61M 39/24, A61M 5/28, A61M 5/34

(54) **DRUG INJECTION DEVICE USING PULSE SHOCK WAVE**
ARZNEIMITTELINJEKTIONSVORRICHTUNG MIT PULSSCHOCKWELLEN
DISPOSITIF D'INJECTION DE MÉDICAMENT AU MOYEN D'ONDE DE CHOC D'IMPULSION

(30) Priority: 14.04.2020 KR 20200045256
(43) Date of publication of application: 08.02.2023
(73) Proprietor: Jeisys Medical Inc., Geumcheon-gu Seoul 08501 (KR)
(72) Inventor: SEO, Kyu Young, Seoul 08501 (KR); YI, Won Ju, Seoul 08501 (KR); KANG, Dong Hwan, Incheon 22229 (KR)
(74) Representative: dompatent von Kreisler Selting Werner - Partnerschaft von Patent- und Rechtsanwälten mbB
(86) International application number: PCT/KR2021/004672
(87) International publication number: WO 2021/210901

(56) References cited:
- KR-A- 20170 027 866
- KR-A- 20180 087 639
- KR-A- 20190 117 919
- KR-A- 20190 127 009
- KR-A- 20190 127 009
- KR-A- 20190 128 338
- KR-A- 20190 128 338
- KR-B1- 101 424 394
- US-A1- 2009 318 859
- US-A1- 2018 056 004

## Description

### [TECHNICAL FIELD]

Embodiments of the inventive concept described herein relate to a drug injection device using pulsed shock waves.

### [BACKGROUND ART]

A drug delivery system is a system designed to efficiently deliver a required amount of drug into the human body by minimizing side effects occurring in an existing method and maximizing therapeutic effects of the drug when using the drug for treatment of a disease or wound in the human body.

An injection method used most commonly in the drug delivery system enables accurate and efficient drug injection, but has problems such as injection phobia due to pain during injection, the risk of infection due to reuse, and generation of a large amount of medical waste.

In order to solve these problems, a drug delivery method such as a needle-free injector has been developed.

For example, a liquid injection technology, which is one of needle-free injection technologies, is a technology that applies shock waves to a liquid through a laser or electric waves to thermally expand the liquid and generate a high-speed liquid stream using the pressure generated at this time to inject the liquid into the skin.

However, the liquid injection technology has a problem in that the shock waves are generated in liquid, so that it is difficult to accurately adjust thermal conductivity (that is, the degree of expansion of the liquid) depending on the density, temperature, and type of the liquid. Furthermore, in the case of using a laser pulse having high energy and a short pulse width in order to generate the shock waves in the liquid, a laser device is required, and therefore the size and price of equipment may be increased. In addition, many optical systems are required to irradiate a laser beam to the liquid, which may result in problems such as damage to the optical systems.

KR20190128338A discloses, a micro-jet injection device using electrical energy to generate bubbles in a fluid within a confined chamber, and these bubbles subsequently drive the delivery of the drug solution through a micro nozzle.

KR20190127009A discloses, a needleless injection device that encompasses a cylindrical main body, a piston, and a driver that heats the first space to pressurize the piston, thereby driving the delivery of drug contained in a separate space through the device's nozzle.

KR101424394B1 discloses a microjet drug delivery device that generates bubbles by concentrating energy onto a pressure generating liquid in a sealed space, which pressurizes a drug-filled space connected by a micro-nozzle, avoiding the need for a pressurized bubble formation.

### [DETAILED DESCRIPTION OF THE INVENTION]

### [TECHINICAL PROBLEM]

Embodiments of the inventive concept provide a drug injection device using pulsed shock waves that easily adjusts the degree of expansion of a liquid, is implemented with small and economical equipment, and prevents damage to an optical system.

### [TECHNICAL SOLUTION]

According to the embodiment of the inventive concept, a drug injection device using pulsed shock waves includes a power unit that generates pulsed power, a pulsed shock wave generating unit that receives the pulsed power and generates the pulsed shock waves, an upper housing in which a liquid and the pulsed shock wave generating unit are disposed therein, a lower housing, connected to the upper housing and that has a drug disposed therein, a shock wave transmitting unit provided between the upper housing and the lower housing and transmitting the shock waves generated in the upper housing to the lower housing, and an injection unit that is disposed in the lower housing and that injects the drug. The pulsed shock wave generating unit includes first and second shock wave generating electrodes that receive the pulsed power and allow a current to instantaneously flow, a shock wave generating unit that generates the pulsed shock waves as the current instantaneously flows between the first and second shock wave generating electrodes, and a first insulating tube, wherein the first shock wave generating electrode is located in contact, or in non-contact within the first insulating tube, and an end of the first shock wave generating electrode inside the first insulating tube that is opposite one end of the second shock wave generating electrode at the closest distance is not exposed outside the first insulating tube.

In addition, according to an embodiment, a drug injection device using pulsed shock waves includes a power unit that operates a voltage charged in a capacitor to a switch and instantaneously generates pulsed power, a pulsed shock wave generating unit that receives the pulsed power and generates the pulsed shock waves, and a housing having a liquid and a drug disposed therein. The liquid is expanded by the pulsed shock waves, and pressure is applied to the drug to inject the drug.

### [ADVANTAGEOUS EFFECTS OF THE INVENTION]

According to an embodiment of the inventive concept, the drug injection device using the pulsed shock waves may easily adjust the degree of expansion of the liquid (e.g., the rate of volume expansion by the gas generated in the liquid), may be implemented with small and economical equipment, and may prevent damage to an optical system.

In addition, microbubble generation and break-down formation may be sequentially performed by providing only a high voltage without needing to apply a low voltage for generating microbubbles and thereafter provide a high voltage for forming break-down, and thus there is an effect that control of the drug injection device is simplified.

### [DESCRIPTION OF THE DRAWINGS]

FIG. 1A is a schematic sectional view illustrating a drug injection device using pulsed shock waves according to an embodiment of the inventive concept.
FIG. 1B is a schematic sectional view of region A of FIG. 1A taken in the -Z direction.
FIG. 2A is a schematic sectional view illustrating the drug injection device using the pulsed shock waves according to an embodiment of the inventive concept.
FIG. 2B is a schematic sectional view of region A of FIG. 2A taken in the -Z direction.
FIG. 2C is a graph depicting voltage/current input waveforms of pulsed power generated from a power unit according to an embodiment of the inventive concept.
FIG. 3 is a schematic sectional view illustrating a drug injection device using pulsed shock waves according to another embodiment of the inventive concept.
FIG. 4 is a schematic view illustrating a first embodiment of a needle unit provided in the drug injection device using the pulsed shock waves according to another embodiment of the inventive concept.
FIG. 5 is a schematic view illustrating a second embodiment of the needle unit provided in the drug injection device using the pulsed shock waves according to another embodiment of the inventive concept.
FIG. 6 is a schematic view illustrating a third embodiment of the needle unit provided in the drug injection device using the pulsed shock waves according to another embodiment of the inventive concept.
FIG. 7 is a schematic view illustrating a fourth embodiment of the needle unit provided in the drug injection device using the pulsed shock waves according to another embodiment of the inventive concept.

### [BEST MODE]

The above and other aspects, features, and advantages of the inventive concept will become apparent from the following description of embodiments given in conjunction with the accompanying drawings. However, the inventive concept is not limited to the embodiments disclosed herein and may be implemented in various different forms. Herein, the embodiments are provided to provide complete disclosure of the inventive concept and to provide thorough understanding of the inventive concept to those skilled in the art to which the inventive concept pertains, and the scope of the inventive concept should be limited only by the accompanying claims**.**

Terms used herein are only for description of embodiments and are not intended to limit the inventive concept. As used herein, the singular forms are intended to include the plural forms as well, unless context clearly indicates otherwise. It will be further understood that the terms "comprise" and/or "comprising" specify the presence of stated features, components, and/or operations, but do not preclude the presence or addition of one or more other features, components, and/or operations. In addition, identical numerals will denote identical components throughout the specification, and the meaning of "and/or" includes each mentioned item and every combination of mentioned items. It will be understood that, although the terms first, second, etc. may be used herein to describe various components, these components should not be limited by these terms. These terms are only used to distinguish one component from another component. Thus, a first component discussed below could be termed a second component without departing from the teachings of the inventive concept.

Unless otherwise defined, all terms (including technical and scientific terms) used herein have the same meaning as commonly understood by those skilled in the art to which the inventive concept pertains. It will be further understood that terms, such as those defined in commonly used dictionaries, should not be interpreted in an idealized or overly formal sense unless expressly so defined herein.

Hereinafter, embodiments of the inventive concept will be described in detail with reference to the accompanying drawings.

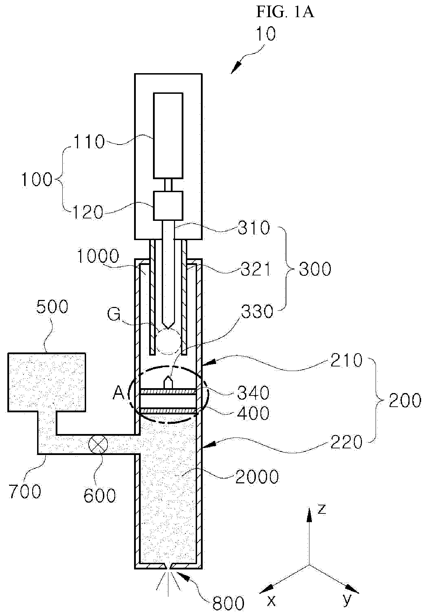

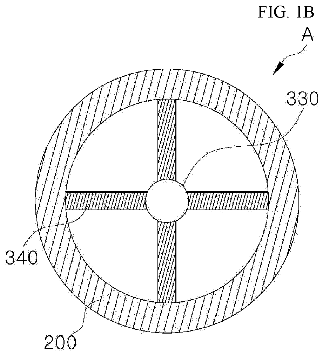

FIG. 1A is a schematic sectional view illustrating a drug injection device using pulsed shock waves according to an embodiment of the inventive concept. FIG. 1B is a schematic sectional view of region A of FIG. 1A taken in the -Z direction.

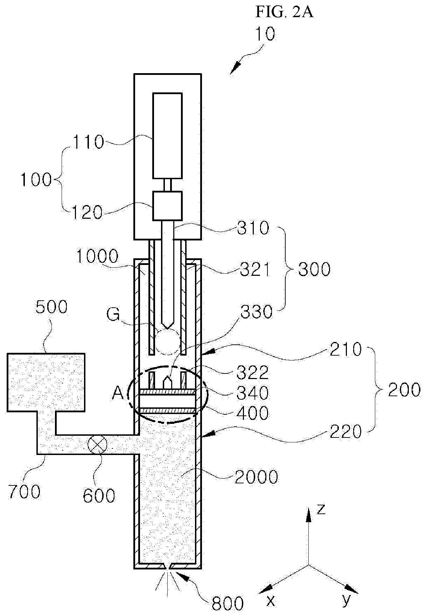

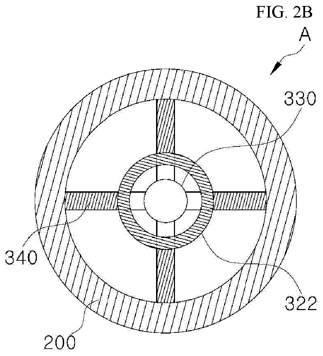

FIG. 2A is a schematic sectional view illustrating the drug injection device using the pulsed shock waves according to an embodiment of the inventive concept. FIG. 2B is a schematic sectional view of region A of FIG. 2A taken in the -Z direction.

Referring to FIGS. 1A, 1B, 2A, and 2B, a drug injection device 10 using the pulsed shock waves according to the embodiment of the inventive concept includes a power unit 100, a pulsed shock wave generating unit 300, and a housing 200. The housing 200 includes an upper housing 210 and a lower housing 220. The drug injection device 10 using the pulsed shock waves according to an embodiment of the inventive concept includes a shock wave transmitting unit 400 and a n injection unit 800.

The power unit 100 instantaneously generates pulsed power by operating a voltage charged in a capacitor as a switch. Although not illustrated, for example, the power unit 100 includes a power supply unit. The power supply unit may preferably be a generator. The generator provides electricity for generating the pulsed power. For example, the generator may boost a low voltage to a high voltage and may generate the pulsed power through the switch.

The power unit 100 may include an electricity storage unit 110 and a switch 120. The electricity storage unit 110 may preferably be at least one selected from a capacitor and an inductor.

In addition, the power unit 100 may further include an electrical circuit that maintains the form of a generated pulse. In this case, the electrical circuit may preferably be a pulse forming network (PFN) and may prevent the form of a square pulse from collapsing due to parasitic inductance, thereby maintaining the form of the pulse.

Electricity generated by the power supply unit may be firstly charged in the electricity storage unit 110. When the switch 120 is turned on, the pulsed power charged in the electricity storage unit 110 may be transmitted to the pulsed shock wave generating unit 300. The switch 120 may supply or cut off electricity. For example, the switch 120 may adjust the rising time of pulsed shock waves by a user.

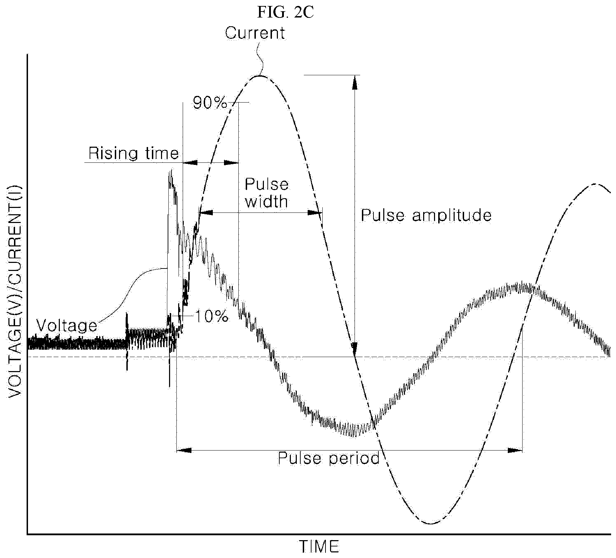

FIG. 2C is a graph depicting voltage/current input waveforms of pulsed power generated from the power unit according to an embodiment of the inventive concept. Here, the horizontal axis of the graph represents passage of time, and the vertical axis of the graph simultaneously represents voltage intensity and current intensity.

As illustrated in FIG. 2C, the pulsed power is to instantaneously increase power by accumulating electrical energy and then emitting a large amount of energy for a very short rising time. Since it is necessary to understand pulse amplitude in order to define the rising time, the pulse amplitude will be described first.

The pulse amplitude indicates the magnitude of a pulse measured at a level at which the pulse remains at a constant value. For example, the pulse amplitude may be expressed as the peak height of the pulse, the effective height of the pulse, or the instantaneous height of the pulse.

The rising time may be time taken from 10% to 90% of the pulse amplitude. For example, the rising time is not particularly limited, but may be in units of several nanoseconds to several milliseconds, and more preferably in units of several nanoseconds to several microseconds.

A pulse width is a time interval for which the amplitude is 1/2 at the rise time and fall time of the pulse.

A pulse period is a period of a pulse signal that is repeated for a unit of time. Here, the unit of time is not particularly limited, but may be 1 second.

Meanwhile, the power unit 100 further includes a generator (not illustrated) for charging the electricity storage unit 110. The generator charges the electricity storage unit by converting an AC voltage into a DC voltage and providing a current to the electricity storage unit. Pulsed power under a specific condition is provided to the pulsed shock wave generating unit by adjusting the switch 120 after the electricity storage unit 110 is charged. That is, the switch 120 provides, to the pulsed shock wave generating unit 300, a voltage raised to a high voltage value within a short period of time (e.g., several microseconds) and maintained at a constant value.

The pulsed shock wave generating unit 300 receives the pulsed power and generates pulsed shock waves. The pulsed shock wave generating unit 300 is disposed in the upper housing 210. The pulsed shock wave generating unit 300 expands a liquid 1000 in the upper housing 210 by generating the pulsed shock waves. The expanded liquid 1000 moves the shock wave transmitting unit 400 in the direction from the upper housing 210 to the lower housing 220 and injects a drug 2000 through the injection unit 800.

The pulsed shock wave generating unit 300 generates the pulsed shock waves.

The pulsed shock wave generating unit 300 may include a cable and may be, for example, a coaxial cable. The cable may maintain low inductance by keeping a short current path. When the cable maintains the low inductance, it may be advantageous for fast pulse generation.

The pulsed shock wave generating unit 300 includes a first shock wave generating electrode and a second shock wave generating electrode, and one or more insulating tubes. The first and second shock wave generating electrodes may receive the pulsed power so that a high voltage may be applied thereto.

Although not illustrated, more shock wave generating electrodes may be included.

Hereinafter, it will be exemplified that one first shock wave generating electrode 310 and one second shock wave generating electrode 330 are provided. However, the inventive concept is not limited thereto, and a plurality of first shock wave generating electrodes 310 and a plurality of second shock wave generating electrodes 330 may be provided.

FIGS. 1A and 2A illustrate one example that the first shock wave generating electrode 310 is connected to the switch 120. However, without being limited thereto, the first shock wave generating electrode 310 may be connected to the switch 120 by a separate connecting unit. The connecting unit may be connected to the first shock wave generating electrode 310 and the second shock wave generating electrode 330, respectively, and may apply a voltage to allow a current to flow.

Insulating tubes 321 and 322 are adjacent to at least one of the shock wave generating electrodes 310 and 330. The insulating tubes 321 and 322 may or may not make contact with at least one of the shock wave generating electrodes 310 and 330. The insulating tubes 321 and 322 include the first insulating tube 321 and the second insulating tube 322.

The first shock wave generating electrode 310 is disposed in the first insulating tube 321. The length of the first insulating tube 321 in the -Z direction is longer than the length of the first shock wave generating electrode 310 in the -Z direction.

The first insulating tube 321 may have various shapes, such as a circular shape, a quadrilateral shape, and the like, when viewed from above, but is not limited thereto.

The first shock wave generating electrode 310 is inserted into the first insulating tube 321. One end of the first shock wave generating electrode 310 is not exposed outside the first insulating tube 321. More specifically, the one end of the first shock wave generating electrode 310 that is opposite one end of the second shock wave generating electrode 330 at the closest distance is not exposed outside the first insulating tube 321.

A shock wave generating unit G generates microbubbles for generation of pulsed shock waves as a current instantaneously flows between the shock wave generating electrodes 310 and 330. The shock wave generating unit G may mean, for example, a region between the first shock wave generating electrode 310 and the first insulating tube 321. The shock wave generating unit G may mean, for example, a region defined by the first shock wave generating electrode 310, the second shock wave generating electrode 330, and the first insulating tube 321.

The second shock wave generating electrode 330 may be connected to a cable 340. The second shock wave generating electrode and the cable may be connected in various manners.

In an embodiment, the liquid 1000 may be disposed between the cable 340 and the shock wave transmitting unit 400. For example, water may be disposed between the cable 340 and the shock wave transmitting unit 400. The shock wave transmitting unit 400 may have the form of a film formed of various materials and, for example, may be an elastic film.

In another embodiment, the second shock wave generating electrode and the cable connected thereto may be coupled to the shock wave transmitting unit 400 having a film form and may be moved toward the lower housing together with a separation film (that is, the shock wave transmitting unit) by liquid expansion. In this case, when the shock wave transmitting unit expands toward the lower housing, only a peripheral region other than the center of the shock wave transmitting unit may be formed to have elasticity, and the second shock wave generating electrode may be disposed at the center of the shock wave transmitting unit. Furthermore, the cable coupled to the shock wave transmitting unit may be stretched together while the shock wave transmitting unit is expanded toward the lower housing by expansion of the upper housing, or may be broken when the shock wave transmitting unit is expanded and then may be shortcircuited again when the shock wave transmitting unit is restored to a normal state.

Although not illustrated, the cable 340 may preferably be connected to the power unit 100.

Although not illustrated, the second shock wave generating electrode 330 may make contact with the shock wave transmitting unit 400.

Furthermore, the second shock wave generating electrode 330 may be disposed on one surface of the upper housing 210. In this case, the second shock wave generating electrode 330 may be disposed on the one surface of the upper housing 210 while being located under one end of the first insulating tube 321, that is, the shock wave generating unit G.

The second shock wave generating electrode 330 may be connected to the cable 340 without the second insulating tube 322, or may be disposed in the second insulating tube 322.

The second insulating tube 322 may have various shapes, such as a circular shape, a quadrilateral shape, and the like, when viewed from above, but is not limited thereto.

For example, referring to FIGS. 1A and 1B, the second shock wave generating electrode 330 may be connected to the cable 340 without being inserted into the second insulating tube (322 of FIGS. 2A and 2B).

For example, referring to FIGS. 2A and 2B, the second shock wave generating electrode 320 may be disposed in the second insulating tube 322. The length of the second insulating tube 322 in the +Z direction may be longer than the length of the second shock wave generating electrode 330 in the +Z direction.

When the second shock wave generating electrode 330 is inserted into the second insulating tube 322, the one end of the second shock wave generating electrode 330 is not exposed outside the second insulating tube 322. More specifically, the one end of the second shock wave generating electrode 330 that is opposite the one end of the first shock wave generating electrode 310 at the closest distance is not exposed outside the second insulating tube 322.

Referring again to FIGS. 1A, 1B, 2A, and 2B, as a specific example, the first shock wave generating electrode 310 inserted into the longer first insulating tube 321 extends in an up/down direction (that is, the Z-axis direction), and the second shock wave generating electrode 330 is disposed in an opposite direction. As the first shock wave generating electrode 310 extends long in the upper housing 210 (that is, a chamber filled with a liquid), the first shock wave generating electrode 310 may extend to a region adjacent to the shock wave transmitting unit 400 separating from the lower housing 220 . Furthermore, the second shock wave generating electrode 330 may be coupled to the shock wave transmitting unit 400. In addition, the first shock wave generating electrode 310 and the second shock wave generating electrode 330 are disposed in opposite directions at a specific distance at which a spark due to a plasma phenomenon is able to be generated when a high voltage is applied.

The pulsed shock wave generating unit 300 of an embodiment of the inventive concept may generate pulsed shock waves by a one-step voltage provision method of directly applying a high voltage for spark generation rather than an existing two-step voltage provision method of providing a low voltage for generating microbubbles and then providing a high voltage for spark generation. This is because when a high voltage is applied in the region of the first insulating tube 321 longer than the one end of the first shock wave generating electrode 310, microbubbles due to a temperature rise may be generated and accordingly, breakdown may occur to generate a spark. That is, a spark may be generated between the first insulating tube 321 longer than the one end of the first shock wave generating electrode 310 and the second shock wave generating electrode 330.

Specifically, in the space inside the first insulating tube 321 at the distal end of the first shock wave generating electrode 310, temperature rises depending on application of a high voltage, and a gas dissolved in the liquid thermally expands to generate microbubbles. As the microbubbles are generated (that is, cavitation occurs in the liquid) within a short period of time, a spark by a plasma phenomenon is generated due to the microbubbles between the first shock wave generating electrode 310 and the second shock wave generating electrode 330 and the applied high voltage, and internal expansion occurs in the upper housing 210.

The housing 200 has a sealed accommodation space. The liquid 1000 and the drug 2000 are disposed in the housing 200. The housing 200 may be divided into the upper housing 210 and the lower housing 220 by the shock wave transmitting unit 400.

The upper housing 210 has a sealed accommodation space. The liquid 1000 is disposed in the upper housing 210. The liquid 1000 may be, for example, water. That is, when the liquid is water, a gas may be dissolved so that microbubbles are able to be generated. However, without being limited thereto, the liquid 1000 may include various liquid materials such as polymer sol and gel, for example, alcohol or polyethylene glycol.

The upper housing 210 may have a schematically cylindrical shape. An upper end of the upper housing 210 may be connected to a transmitting unit. The shock wave transmitting unit 400 may be disposed at a lower end of the upper housing 210.

The volume of the liquid 1000 disposed in the upper housing 210 may be expanded by pulsed shock waves. When the volume of the liquid 1000 is increased by the pulsed shock waves, the pressure in the upper housing 210 is increased.

The lower housing 220 has a sealed accommodation space. The drug 2000 is disposed in the lower housing 220. The lower housing 220 may have a schematically cylindrical shape. The shock wave transmitting unit 400 may be disposed at an upper end of the lower housing 220. A lower end of the lower housing 220 may be connected to the injection unit 800. One side of the lower housing 220 may be connected to a drug delivery unit 700.

When the pressure in the upper housing 210 is increased, pressure is applied to the inside of the lower housing 220. That is, the pressure in the lower housing 220 may be increased. Accordingly, the pressure may be applied to the drug 2000. The drug 2000 may be injected through the injection unit 800 and then injected into a user. A detailed description thereof will be given below.

The shock wave transmitting unit 400 is provided between the upper housing 210 and the lower housing 220. The shock wave transmitting unit 400 divides the housing 200 into the upper housing 210 and the lower housing 220.

The shock wave transmitting unit 400 separates the upper housing 210 and the lower housing 220. One surface of the upper housing 210 and one surface of the lower housing 220 are formed by the shock wave transmitting unit 400. Accordingly, the expansion of the liquid 1000 disposed in the upper housing 210 may cause an increase in the pressure in the lower housing 220 through deformation of the shock wave transmitting unit 400.

The shock wave transmitting unit 400 is not changed in quality or damaged by pulsed shock waves. The shock wave transmitting unit 400 does not absorb pulsed shock waves and is vibrated by the pulsed shock waves. The shock wave transmitting unit 400 has elasticity. The shock wave transmitting unit 400 transmits only the pressure generated by the increase in the volume of the liquid 1000 to the inside of the lower housing 220. The shock wave transmitting unit 400 transmits only the pressure generated by the increase in the volume of the liquid 1000 to the drug 2000 in the lower housing 220. The shock wave transmitting unit 400 blocks penetration of the liquid 1000 and the drug 2000, heat transfer, and the like.

The shock wave transmitting unit 400 may be formed of, for example, natural rubber or synthetic rubber harmless to the human body.

Furthermore, when the shock wave transmitting unit 400 includes the second shock wave generating electrode 321, the second shock wave generating electrode 321 may be disposed at the center of the shock wave transmitting unit 400, and a conductive wire extending from the second shock wave generating electrode 321 may be included. As the region surrounding the second shock wave generating electrode 321 of the shock wave transmitting unit 400 has elasticity, it may be restored after being stretched by the increase in the pressure in the upper housing 210.

The injection unit 800 is disposed in the lower housing 220 as a injection nozzle. For example, the injection unit 800 may be defined in the form of a hole at the lower end of the lower housing 220. However, the inventive concept is not limited thereto, and if the injection unit 800 is able to inject the drug, the injection unit 800 may be connected to the lower housing 220 and may protrude in the direction from the upper end to the lower end of the lower housing 220. The injection unit 800 injects the drug 2000. The injection unit 800 may inject the drug 2000 in the Z-axis direction.

Furthermore, the speed at which the drug is injected is determined based on the diameter of the injection unit 800. That is, if the injection speed is low, the drug may not be injected into a skin, and therefore the injection unit 800 may be implemented to have a nozzle diameter by which the drug is injected at an appropriate speed based on the pressure transmitted from the upper housing 210 to the lower housing 220.

For example, the injection unit 800 may have a diameter of 50 micrometers to 1000 micrometers. When the diameter of the injection unit 800 is less than 50 micrometers, the amount of the injected drug 2000 may be less, and the drug 2000 may not be injected to a sufficient depth into the body of the user into which the drug 2000 is injected. When the diameter of the injection unit 800 exceeds 1000 micrometers, the injected microjet may have a large diameter, and therefore the amount of the drug 2000 bounced off the surface of the skin may be increased so that waste of the drug 2000 may become severe. The injection unit 800 may inject the drug 2000 in the Z-axis direction. In this specification, the "Z-axis direction" means the direction of an axis orthogonal to the X-axis direction (horizontal direction) and the Y-axis direction (vertical direction) in a three-dimensional coordinate system. More specifically, the injection unit 800 may inject the drug 200 in the direction from the upper housing 210 to the lower housing 220.

When the volume of the liquid 1000 is increased by the pulsed shock waves applied to the liquid 1000 as mentioned above, the pressure in the upper housing 210 is increased, and pressure is applied to the inside of the lower housing 220. Accordingly, the pressure may be applied to the drug 2000, and the pressurized drug 2000 may be injected into a userthrough the injection unit 800.

The drug injection device 10 using the pulsed shock waves according to an embodiment of the inventive concept may further include a drug storage unit 500, the drug delivery unit 700, and a check valve 600.

The drug storage unit 500 stores the drug 2000 to be provided to the lower housing 220. For example, the drug storage unit 500 may be disposed on a side surface of the lower housing 220.

The drug delivery unit 700 receives the drug 2000 from the drug storage unit 500 and provides the drug 2000 to the lower housing 220. For example, the drug delivery unit 700 may be connected to a side surface of the lower housing 220.

The check valve 600 allows the drug 2000 to be delivered only in the direction from the drug storage unit 500 to the lower housing 220. For example, the check valve 600 prevents the drug 2000 from being delivered in the direction from the lower housing 220 to the drug storage unit 500. For example, the check valve 600 may be disposed in the drug delivery unit 700.

Furthermore, the drug injection device 10 using pulsed shock waves according to another embodiment of the inventive concept further includes a liquid circulation unit (not illustrated). The liquid circulation unit serves to circulate a liquid in an upper housing. As microbubbles are generated and a spark due to a plasma phenomenon is generated, the amount of gas dissolved in the liquid may be decreased, and the pressure in the upper housing 210 may be increased by the generated gas. Accordingly, the liquid circulation unit may circulate the liquid in the upper housing 210 to fill the upper housing 210 with the liquid capable of generating appropriate pressure. Thus, drug injection of the drug injection device 10 may be constantly performed.

Specifically, the liquid circulation unit may include a solenoid valve and may circulate and change the liquid in the upper housing 210 by opening the solenoid valve as needed.

Moreover, the drug injection device 10 using pulsed shock waves according to another embodiment of the inventive concept further includes a pressure sensor (not illustrated). The pressure sensor serves to measure the pressure before and after a spark is generated and when the spark is generated. To this end, the pressure sensor may be disposed at a specific position in the upper housing 210.

For example, the pressure sensor may detect that the pressure in the upper housing 210 is raised to a reference value or more and may circulate a liquid in the upper housing 210 by driving a liquid circulation unit such that the shock wave transmitting unit 400 is in an equilibrium state. In addition, the pressure sensor measures the pressure generated when the spark is generated, and when the measurement value of the pressure sensor is not greater than or equal to the reference value during operation, a controller (not illustrated) determines that the gas dissolved in the liquid is too little and circulates the liquid in the upper housing 210.

Hereinafter, a method of injecting the drug 2000 to a user by using the drug injection device 10 using the pulsed shock waves according to an embodiment of the inventive concept will be described in brief.

When the power unit 100 generates pulsed power, the pulsed shock wave generating unit 300 receives the pulsed power and generates pulsed shock waves. When the pulsed shock waves are generated, the volume of the liquid 1000 provided in the upper housing 210 is expanded. As the volume of the liquid 1000 is expanded, the pressure in the upper housing 210 is increased. As the pressure in the upper housing 210 is increased, the shock wave transmitting unit 400, which has elasticity, transmits the increased pressure into the lower housing 220. At this time, the shock wave transmitting unit 400 is not damaged by the pressure. When the increased pressure in the upper housing 210 is provided to the inside of the lower housing 220, the drug 2000 may be injected into a user through the injection unit 800. When the drug 2000 is additionally required in the lower housing 220, the check valve 600 may be opened, and the drug 2000 may be provided from the drug storage unit 500 into the lower housing 220.

The drug injection device 10 using the pulsed shock waves according to an embodiment of the inventive concept may adjust the rising time from nanoseconds to milliseconds through the power unit 100 that generates the pulsed power and thus may generate short shock waves. Due to this, the liquid may be thermally expanded within a short period of time, and the drug may be injected into a user at high speed.

Further, the drug injection device 10 may adjust the injection amount of the drug in the lower housing 220 by adjusting the intensity of the pressure generated in the upper housing 210 by the generated pulsed power. Accordingly, a user may inject the drug in a desired amount, and thus the drug may be prevented from being wasted.

Furthermore, by implementing an electric shock wave drug injection device that solves the problem in which a low voltage has to be first provided to generate microbubbles, drug injection may be more rapidly performed.

Moreover, the drug injection device 10 using the pulsed shock waves according to an embodiment of the inventive concept uses pulsed power rather than a laser. Accordingly, a problem occurring when the laser is used, more specifically, a large device structure and expensive facility cost are not required. In addition, since an optical part through which a laser beam passes is not required, the drug injection device 10 may fundamentally solve a problem caused by the optical part, for example, a problem in which a cable arrangement state between a main body and a needle-free injector has to be limited to accurately transmit a laser from the main body generating the laser to the needle-free injector (e.g., a handpiece unit injecting a drug).

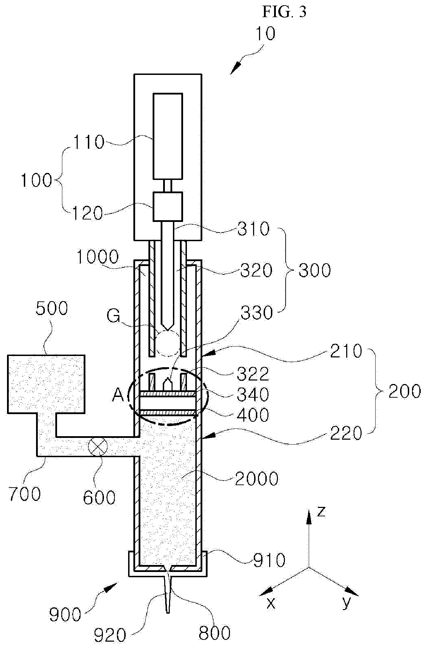

FIG. 3 is a schematic sectional view illustrating a drug injection device using pulsed shock waves according to another embodiment of the inventive concept.

As illustrated in FIG. 3, the drug injection device using the pulsed shock waves according to another embodiment of the inventive concept may further include a needle adaptor 900.

The needle adapter 900 may be detachably coupled to a lower housing so as to be fluidly coupled with an injection unit 800 and may inject a drug into a skin.

The needle adaptor 900 may include an adaptor body 910 and a needle unit 920.

The adaptor body 910 may be attached to and detached from the lower housing 220. The adaptor body 910 may be formed in a shape surrounding a region in which the injection unit 800 of the lower housing 220 is provided.

The needle unit 920 may be connected to the adaptor body 910 and may be inserted into the skin to inject the drug introduced from the injection unit 800. In more detail, the needle unit 920 may be inserted into a deep part of the skin to a set depth and then may inject the drug, which is subjected to strong pressure by pulsed shock waves from the injection unit 800, at high speed.

At this time, the depth to which the drug is inserted into the deep part of the skin may be accurately adjusted by adjusting the depth to which the drug is injected into the deep part of the skin. Here, a user may manually adjust the depth to which the needle unit 920 is inserted into the deep part of the skin.

Hereinafter, various embodiments of the needle unit 920 will be described.

The needle unit 920 may be at least one of a needle 921, a porous needle 922, a cannula 923, and a porous cannula 924.

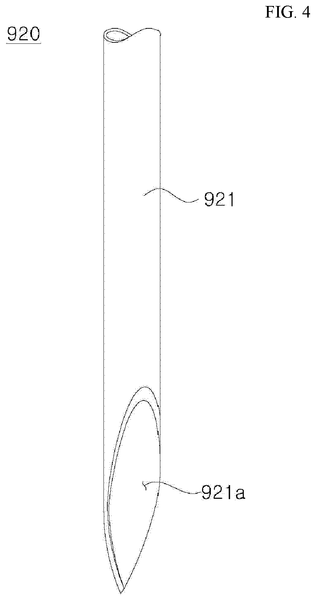

FIG. 4 is a schematic view illustrating a first embodiment of the needle unit provided in the drug injection device using the pulsed shock waves according to another embodiment of the inventive concept.

As illustrated in FIG. 4, the first embodiment of the needle unit 920 may be the needle 921.

The needle 921 may protrude from the adaptor body 910 in an up/down direction, and a flow passage connected to the lower housing 220 may be formed in the needle 921.

A distal end of the needle 921 is formed in a pointed shape. Accordingly, due to the pointed shape of the distal end of the needle 921, the needle 921 may be easily inserted into a deep part of the skin.

A needle hole 921a through which the drug is ejected may be formed at the distal end of the needle 921. The needle hole 921a may be formed in the axial direction of the needle 921.

In this embodiment, after the needle 921 is inserted into the deep part of the skin, the drug subjected to strong pressure by pulsed shock waves may be injected into a single portion of the deep part of the skin along the needle hole 921a. Accordingly, in this embodiment, the drug may be injected only into a required portion of the deep part of the skin, and thus it is possible to perform detailed treatment.

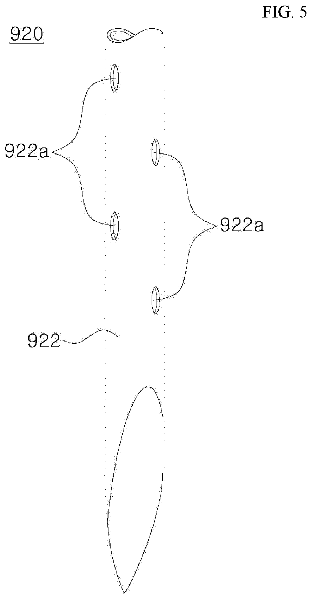

FIG. 5 is a schematic view illustrating a second embodiment of the needle unit provided in the drug injection device using the pulsed shock waves according to another embodiment of the inventive concept.

As illustrated in FIG. 5, the second embodiment of the needle unit 920 may be the porous needle 922.

The porous needle 922 may protrude from the adaptor body 910 in an up/down direction, and a flow passage connected to the lower housing 220 may be formed in the porous needle 922.

A distal end of the porous needle 922 is formed in a pointed shape. Accordingly, due to the pointed shape of the distal end of the porous needle 921, the porous needle 921 may be easily inserted into a deep part of the skin.

A plurality of porous needle holes 922a may be formed in an outer circumferential surface of the porous needle 922. The plurality of porous needle holes 922a may be radially formed in the outer circumferential surface of the porous needle 922.

In this embodiment, after the porous needle 922 is inserted into the deep part of the skin, the drug subjected to strong pressure by pulsed shock waves may be injected into a plurality of portions of the deep part of the skin along the plurality of porous needle hole 922a. Accordingly, in this embodiment, the drug may be injected into various portions of the deep part of the skin.

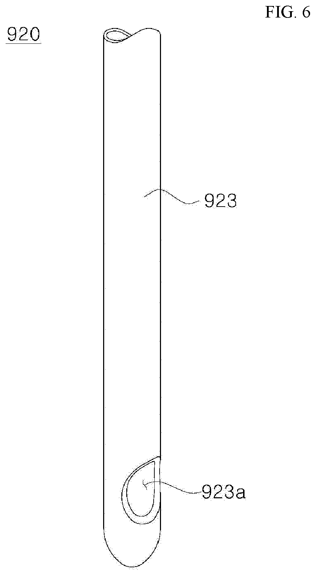

FIG. 6 is a schematic view illustrating a third embodiment of the needle unit provided in the drug injection device using the pulsed shock waves according to another embodiment of the inventive concept.

As illustrated in FIG. 6, the third embodiment of the needle unit 920 may be the cannula 923.

The cannula 923 may protrude from the adaptor body 910 in an up/down direction, and a flow passage connected to the lower housing 220 may be formed in the cannula 923.

A distal end of the cannula 923 is formed in a circular shape. Accordingly, due to the circular shape of the distal end of the cannula 923, a risk of damaging a blood vessel may be reduced when the cannula 923 is inserted into a deep part of the skin.

A cannula hole 923a may be formed in an outer circumferential surface of the cannula 923.

In this embodiment, after the cannula 923 is inserted into the deep part of the skin, the drug subjected to strong pressure by pulsed shock waves may be injected into a relatively wide region of the deep part of the skin along an outer circumferential surface of the cannula hole 923a. At this time, an operator may inject the drug into the deep part of the skin in various directions by turning the cannula 923.

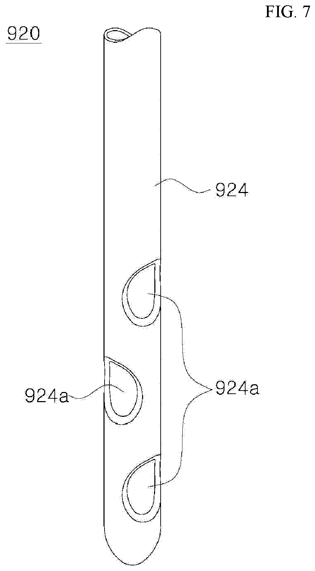

FIG. 7 is a schematic view illustrating a fourth embodiment of the needle unit provided in the drug injection device using the pulsed shock waves according to another embodiment of the inventive concept.

As illustrated in FIG. 7, the fourth embodiment of the needle unit 920 may be the porous cannula 924.

The porous cannula 924 may protrude from the adaptor body 910 in an up/down direction, and a flow passage connected to the lower housing 220 may be formed in the porous cannula 924.

A distal end of the porous cannula 924 is formed in a circular shape. Accordingly, due to the circular shape of the distal end of the porous cannula 924, a risk of damaging a blood vessel may be reduced when the porous cannula 924 is inserted into a deep part of the skin.

A plurality of porous cannula holes 924a may be formed in an outer circumferential surface of the porous cannula 924. The plurality of porous cannula holes 924a may be radially formed in the outer circumferential surface of the porous cannula 924.

In this embodiment, after the porous cannula 924 is inserted into the deep part of the skin, the drug subjected to strong pressure by pulsed shock waves may be injected into a plurality of portions of the deep part of the skin along the plurality of porous cannula holes 924a. Accordingly, in this embodiment, the drug may be injected into various portions of the deep part of the skin.

According to an embodiment of the inventive concept, after at least one of one or more needles, one or more porous needles, one or more cannulas, and one or more porous cannulas are inserted into a deep part of the skin to a set depth, the drug subjected to strong pressure by pulsed shock waves may be injected at high speed.

While the inventive concept has been described with reference to embodiments, it will be apparent to those skilled in the art that various changes and modifications may be made without departing from the scope of the claimed invention as defined by the appended independent claim. Therefore, it should be understood that the above embodiments are not limiting, but illustrative.

## Claims

1. A drug injection device (10) using pulsed shock waves, the drug injection device (10) comprising:
a power unit (100) configured to generate pulsed power;
a pulsed shock wave generating unit (300) configured to receive the pulsed power and generate the pulsed shock waves;
an upper housing (210) in which a liquid (1000) and the pulsed shock wave generating unit (300) are disposed therein;
a lower housing (220) connected to the upper housing (210) and having a drug (2000) disposed therein;
a needle adaptor detachably coupled to the lower housing (220);
a shock wave transmitting unit (400) provided between the upper housing (210) and the lower housing (220) and configured to transmit the shock waves generated in the upper housing (210) to the lower housing (220); and
an injection unit (800) disposed in the lower housing (220) and configured to inject the drug (2000),
wherein the pulsed shock wave generating unit (300) includes:
a first shock wave generating electrode (310) and a second shock wave generating electrode (330) configured to receive the pulsed power (100) and allow a current to flow; and
a first insulating tube (321), wherein the first shock wave generating electrode (310) is located in contact, or in non-contact within the first insulating tube (321)
**characterized in that** an end of the first shock wave generating electrode inside the first insulating tube that is opposite one end of the second shock wave generating electrode (330) at the closest distance is not exposed outside the first insulating tube (321).

2. The drug injection device (10) of claim 1, wherein the power unit (100) includes:
a power supply unit (100) configured to supply a voltage and a current;
an electricity storage unit (110) configured to store electricity supplied from the power supply unit (100); and
a switch (120) configured to apply stored electrical energy as pulsed power from the electricity storage unit. (110).

3. The drug injection device (10) of claim 2, wherein the power unit (100) further includes an electrical circuit configured to maintain a form of a generated pulse.

4. The drug injection device (10) of claim 1, wherein the first insulating tube (321) having the first shock wave generating electrode (310) located therein is longer than the first shock wave generating electrode (310).

5. The drug injection device (10) of claim 4, further including a second insulating tube (322) having the second shock wave generating electrode (330) located therein, wherein the second insulating tube (322) is longer than the second shock wave generating electrode (330).

6. The drug injection device (10) of claim 1, wherein the pulsed shock wave generating unit (300) includes a cable (340) configured to connect the power unit (100) and the second shock wave generating electrode (330).

7. The drug injection device (10) of claim 1, wherein when bubbles generated from the pulsed shock wave generating unit (300) increase pressure in the upper housing (210), the shock wave transmitting unit (400) transmits the increased pressure to the lower housing (220).

8. The drug injection device (10) of claim 1, wherein the injection unit (800) is configured to inject the drug (2000) when increased pressure in the upper housing (210) is transmitted to the lower housing (220).

9. The drug injection device (10) of claim 1, further comprising:
a drug storage unit (500) configured to store the drug (2000) to be provided to the lower housing (220); and
a check valve (600) configured to allow the drug (2000) to be delivered only in a direction from the drug storage unit (500) to the lower housing (220).

10. The drug injection device (10) of claim 1, wherein the needle adaptor includes:
an adaptor body attached to and detached from the lower housing (220); and
a needle unit configured to protrude from the adaptor body and inserted into the skin to inject the drug (2000) introduced from the injection unit (800).

11. The drug injection device (10) of claim 10, wherein the needle unit is at least one of one or more needles, one or more porous needles, one or more cannulas, and one or more porous cannulas.

## Patentansprüche

1. Arzneimittelinjektionsvorrichtung (10), die Pulsschockwelle verwendet, wobei die Arzneimittelinjektionsvorrichtung (10) aufweist:
eine Leistungseinheit (100), die dazu ausgebildet ist, eine Pulsleistung zu erzeugen;
eine Pulsschockwellen-Erzeugungseinheit (300), die dazu ausgebildet ist, die Pulsleistung zu empfangen und die Pulsschockwellen zu erzeugen;
ein oberes Gehäuse (210), in dem eine Flüssigkeit (1000) und die Pulsschockwellen-Erzeugungseinheit (300) angeordnet sind;
ein unteres Gehäuse (220), das mit dem oberen Gehäuse (210) verbunden ist und in dem sich ein Arzneimittel (2000) befindet;
einen Nadeladapter, der lösbar mit dem unteren Gehäuse (220) verbunden ist;
eine Schockwellen-Übertragungseinheit (400), die zwischen dem oberen Gehäuse (210) und dem unteren Gehäuse (220) vorgesehen und dazu ausgebildet ist, die in dem oberen Gehäuse (210) erzeugten Schockwellen an das untere Gehäuse (220) zu übertragen; und
eine Injektionseinheit (800), die in dem unteren Gehäuse (220) angeordnet und dazu ausgebildet ist, das Arzneimittel (2000) zu injizieren,
wobei die Pulsschockwellen-Erzeugungseinheit (300) aufweist:
eine erste Schockwellen-Erzeugungselektrode (310) und eine zweite Schockwellen-Erzeugungselektrode (330), die dazu ausgebildet ist, die Pulsleistung (100) zu empfangen und einen Stromfluss zu ermöglichen; und
einer ersten Isolierröhre (321), wobei sich die erste Schockwellen-Erzeugungselektrode (310) in Kontakt oder in Nicht-Kontakt innerhalb der ersten Isolierröhre (321) befindet,
**dadurch gekennzeichnet, dass** ein Ende der ersten Schockwellen-Erzeugungselektrode in der ersten Isolierröhre, das entgegengesetzt zu einem Ende der zweiten Schockwellen-Erzeugungselektrode (330) in geringstem Abstand ist, nicht außerhalb der ersten Isolierröhre (321) freiliegt.

2. Arzneimittelinjektionsvorrichtung (10) nach Anspruch 1, wobei die Leistungseinheit (100) aufweist:
eine Stromversorgungseinheit (100), die dazu ausgebildet ist, eine Spannung und einen Strom zuzuführen;
eine Stromspeichereinheit (110), die dazu ausgebildet ist, von der Stromversorgungseinheit (100) zugeführten Strom zu speichern; und
einen Schalter (120), der dazu ausgebildet ist, gespeicherte elektrische Energie als Pulsleistung von der Stromspeichereinheit anzulegen (110).

3. Arzneimittelinjektionsvorrichtung (10) nach Anspruch 2, wobei die Leistungseinheit (100) ferner eine elektrische Schaltung aufweist, die dazu ausgebildet ist, eine Form eines erzeugten Pulses aufrechtzuerhalten.

4. Arzneimittelinjektionsvorrichtung (10) nach Anspruch 1, wobei die erste Isolierröhre (321), in der sich die erste Schockwellen-Erzeugungselektrode (310) befindet, länger ist als die erste Schockwellen-Erzeugungselektrode (310).

5. Arzneimittelinjektionsvorrichtung (10) nach Anspruch 4, die ferner eine zweiten Isolierröhre (322) aufweist, in der sich die zweite Schockwellen-Erzeugungselektrode (330) befindet, wobei die zweite Isolierröhre (322) länger als die zweite Schockwellen-Erzeugungselektrode (330) ist.

6. Arzneimittelinjektionsvorrichtung (10) nach Anspruch 1, wobei die Pulsschockwellen-Erzeugungseinheit (300) ein Kabel (340) aufweist, das dazu ausgebildet ist, die Leistungseinheit (100) und die zweite Schockwellen-Erzeugungselektrode (330) anzuschließen.

7. Arzneimittelinjektionsvorrichtung (10) nach Anspruch 1, wobei, wenn von der Pulsschockwellen-Erzeugungseinheit (300) erzeugte Blasen den Druck im oberen Gehäuse (210) erhöhen, überträgt die Schockwellen-Übertragungseinheit (400) den erhöhten Druck an das untere Gehäuse (220).

8. Arzneimittelinjektionsvorrichtung (10) nach Anspruch 1, wobei die Injektionseinheit (800) dazu ausgebildet ist, das Arzneimittel zu injizieren, wenn der erhöhte Druck in dem oberen Gehäuse (210) an das untere Gehäuse (220) übertragen wird.

9. Arzneimittelinjektionsvorrichtung (10) nach Anspruch 1, die ferner aufweist:
eine Arzneimittelspeichereinheit (500), die dazu ausgebildet ist, das Arzneimittel (2000) zu speichern, das dem unteren Gehäuse (220) zugeführt werden soll; und ein Rückschlagventil (600), das dazu ausgebildet ist, zu ermöglichen, dass das Arzneimittel (2000) nur in einer Richtung von der Arzneimittelspeichereinheit (500) an das untere Gehäuse (220) abgegeben wird.

10. Arzneimittelinjektionsvorrichtung (10) nach Anspruch 1, wobei der Nadeladapter aufweist:
einen Adapterkörper, der an dem unteren Gehäuse (220) angebracht und von diesem gelöst wird; und
eine Nadeleinheit, die dazu ausgebildet ist, von dem Adapterkörper vorzustehen und in die Haut eingeführt zu werden, um das von der Injektionseinheit (800) eingeführte Arzneimittel (2000) zu injizieren.

11. Arzneimittelinjektionsvorrichtung (10) nach Anspruch 10, wobei die Nadeleinheit mindestens eines aus einer oder mehreren Nadeln, einer oder mehreren durchlässigen Nadeln, einer oder mehrere Kanülen, und einer oder mehreren durchlässigen Kanülen ist.

## Revendications

1. Dispositif d'injection de médicament (10) utilisant des ondes de choc pulsées, le dispositif d'injection de médicament (10) comprenant :
une unité de puissance (100) configurée pour générer de la puissance pulsée ;
une unité de génération d'ondes de choc pulsées (300) configurée pour recevoir la puissance pulsée et générer les ondes de choc pulsées ;
un logement supérieur (210) dans lequel un liquide (1000) et l'unité de génération d'ondes de choc pulsées (300) sont disposés à l'intérieur de celui-ci ;
un logement inférieur (220) raccordé au logement supérieur (210) et ayant un médicament (2000) disposé à l'intérieur de celui-ci ;
un adaptateur d'aiguille accouplé de façon détachable au logement inférieur (220) ;
une unité de transmission d'ondes de choc (400) fournie entre le logement supérieur (210) et le logement inférieur (220) et configurée pour transmettre les ondes de choc générées dans le logement supérieur (210) au logement inférieur (220) ; et
une unité d'injection (800) disposée dans le logement inférieur (220) et conçue pour injecter le médicament (2000),
dans lequel l'unité de génération d'ondes de choc pulsées (300) comporte :
une première électrode de génération d'ondes de choc (310) et une seconde électrode de génération d'ondes de choc (330) configurées pour recevoir la puissance pulsée (100) et permettre à un courant de circuler ; et
un premier tube isolant (321), dans lequel la première électrode de génération d'ondes de choc (310) est localisée en contact, ou sans contact au sein du premier tube isolant (321) **caractérisé en ce qu'**une extrémité de la première électrode de génération d'ondes de choc à l'intérieur du premier tube isolant qui est opposée à une extrémité de la seconde électrode de génération d'ondes de choc (330) à la distance la plus proche n'est pas exposée à l'extérieur du premier tube isolant (321).

2. Dispositif d'injection de médicament (10) selon la revendication 1, dans lequel l'unité de puissance (100) comporte :
une unité d'alimentation en puissance (100) configurée pour alimenter une tension et un courant ;
une unité de stockage d'électricité (110) configurée pour stocker de l'électricité alimentée à partir de l'unité d'alimentation en puissance (100) ; et
un commutateur (120) configuré pour appliquer l'énergie électrique stockée en tant que puissance pulsée à partir de l'unité de stockage d'électricité. (110).

3. Dispositif d'injection de médicament (10) selon la revendication 2, dans lequel l'unité de puissance (100) comporte en outre un circuit électrique configuré pour maintenir une forme d'une impulsion générée.

4. Dispositif d'injection de médicament (10) selon la revendication 1,
dans lequel le premier tube isolant (321) ayant la première électrode de génération d'ondes de choc (310) localisée à l'intérieur de celui-ci est plus long que la première électrode de génération d'ondes de choc (310).

5. Dispositif d'injection de médicament (10) selon la revendication 4, comportant en outre un second tube isolant (322) ayant la seconde électrode de génération d'ondes de choc (330) localisée à l'intérieur de celui-ci, dans lequel le second tube isolant (322) est plus long que la seconde électrode de génération d'ondes de choc (330).

6. Dispositif d'injection de médicament (10) selon la revendication 1, dans lequel l'unité de génération d'ondes de choc pulsées (300) comporte un câble (340) configuré pour connecter l'unité de puissance (100) et la seconde électrode de génération d'ondes de choc (330).

7. Dispositif d'injection de médicament (10) selon la revendication 1, dans lequel lorsque des bulles générées à partir de l'unité de génération d'ondes de choc pulsées (300) augmentent la pression dans le logement supérieur (210), l'unité de transmission d'ondes de choc (400) transmet la pression augmentée au logement inférieur (220).

8. Dispositif d'injection de médicament (10) selon la revendication 1, dans lequel l'unité d'injection (800) est conçue pour injecter le médicament (2000) lorsqu'une pression augmentée dans le logement supérieur (210) est transmise au logement inférieur (220).

9. Dispositif d'injection de médicament (10) selon la revendication 1, comprenant en outre :
une unité de stockage de médicament (500) conçue pour stocker le médicament (2000) à fournir au logement inférieur (220) ; et
un clapet antiretour (600) conçu pour permettre au médicament (2000) d'être délivré uniquement dans une direction allant de l'unité de stockage de médicament (500) au logement inférieur (220).

10. Dispositif d'injection de médicament (10) selon la revendication 1, dans lequel l'adaptateur d'aiguille comporte :
un corps d'adaptateur fixé au et détaché du logement inférieur (220) ; et
une unité d'aiguille conçue pour faire saillie du corps d'adaptateur et insérée dans la peau pour injecter le médicament (2000) introduit à partir de l'unité d'injection (800).

11. Dispositif d'injection de médicament (10) selon la revendication 10, dans lequel l'unité d'aiguille est au moins l'une parmi une ou plusieurs aiguilles, une ou plusieurs aiguilles poreuses, une ou plusieurs canules et une ou plusieurs canules poreuses.
